# EUROPEAN PATENT APPLICATION

(11) **EP 1 216 672 A1**
(43) Date of publication of application: **26.06.2002**
(21) Application number: 00127792.0
(22) Date of filing: 19.12.2000
(51) Int. Cl.: A61F 13/42

(54) **Indicator means for use with an absorbent product**

(71) Applicant: SCA Hygiene Products AB, 405 03 Göteborg (SE)
(72) Inventor: Kling, Robert, 511 63 Skene (SE)
(74) Representative: Harrison, Michael Charles

(57) **Abstract**

An indicator means (13) for an absorbent product (1) such as a diaper has an indicator portion (6) at one end. The indicator portion (6) is connected to a transport means (5) having the form of one or more hollow fibres. The hollow fibre has one of its ends coupled to the indicator portion (6). Fluids or ions can be transported in the fibre capillary so as to indicate a condition such as wetness or pH within a diaper (1).

## Description

### Field of the invention:

The present invention relates to an indicator means for use with an absorbent product of the type intended for the absorption of bodily exudates, such as diapers or other absorbent garments of a disposable or re-usable type. In particular, the invention relates to the structure of a transport means which is coupled to the indicator means.

### Background to the invention:

Visual indicator means in absorbent products are known in the art, whereby the presence of exudates can be made apparent to an observer. One example is disclosed in WO-A-99/23985 which employs fibre optic strands having one end within an absorbent core and an opposite end adjacent the back sheet of the product. In this way, an attendant can view whether the product is soiled or clean without inspecting the absorbent product internally. This system relies however on the attendant being able to distinguish between dark and light areas which anyway might or might not be attributable to exudates.

It is also known from US-A-3 794 024 to provide a visual indicator not using fibre optics. This prior art indicator is aimed at informing the user of the extent to which a tampon has residual absorbent capacity. The indicator uses a collected bundle of threads formed into a string to act as a wick. By means of the capillaries between the various threads, menses is drawn towards one end of the string and can be displayed at that location. Alternatively, the menses can be used to trigger a further indicator such as a temperature change indicator positioned within the vagina.

A further wick system in a diaper is known from US-A-4 114 621, in which a wick runs through the entire length of the diaper and is coated with an indicator substance. The indicator substance changes colour on contact with urine.

Whilst wick systems transport fluids, the speed with which transport is effected often varies greatly and is generally an unreliable means of transport. This is largely due to the difficulty in controlling and/or maintaining the capillary pressure in the wicks due to variations in tightness of the thread windings in the string and the variation in the uniformity of the threads themselves. Such variations exist between different strings and even along the length of a single string. These factors and others can vastly alter the capillary pressure and thus the reliability and speed with which fluid can be transported.

DE 29822846-U1 discloses a further indicator means using a detector portion in the core of an absorbent product which communicates by means of an electrical conductor with an indicator portion (a buzzer) at the waist band of an absorbent product. When urination occurs, the buzzer is sounded. Such a system is relatively costly and requires electrical elements with a power source. Circuit connection difficulties and the like also make such devices not only difficult to use but often unreliable. Thus, such indicators are less suitable for disposable products.

Document US-A-4 231 370 discloses a pH indicator coating strip in an absorbent product to detect wetness by urine. Wetness at all urine pH values can be detected with differing degrees of speed, as a result of the direct action of the exudate on the indicator. Such a design suffers from difficulties of visibility since the indicator portion is activated substantially at the point of exudate release. The device also suffers from a lack of selectivity in the pH values detected and the time at which an indication is given cannot be controlled in a predetermined manner.

The soiling of diapers and certain other absorbent products is perhaps most commonly due to the presence of urine and/or faeces in such articles. In particular when the presence of faeces is detected, the product should be removed and replaced by a clean one. The soiled product may be discarded if it is of the disposable type, or it can be cleaned and used again if it is of the reusable type.

With infants, diapers are changed typically between four and eight times during a twenty-four hour period. With adults, the frequency of changing incontinence garments such as diapers is often left to the adult's discretion based on a level of comfort or convenience, although when the wearer is bed-ridden, changing frequency often depends on the attendant personnel. Diapers in particular are often not changed at an appropriate time, when viewed in terms of the damage that may occur to a wearer's skin not only by the presence of urine alone, but in particular when urine and faeces are together in the diaper. This condition may often remain undetected for several hours, especially when the wearer exhibits no immediate signs of discomfort.

With babies for example, whose skin is particularly sensitive, diapers are often changed when the baby cries or otherwise indicates discomfort, or upon specifically checking the diaper for the presence of excess wetness and/or faeces. The checking process itself may often be misleading however, since an attendant often relies on an inspection by stretching open one leg elastic only, such that some faecal and urine discharges often go undetected.

Whilst urine is typically quickly absorbed and often held separate from the wearer's skin in an absorbent core, faeces is generally maintained against the skin since it does not pass through into the core, unless special provision is made for this in the top sheet design. When faeces is present first and then followed by urine, the urine itself may also be held in contact with the skin by the faeces. The presence of urine and faeces together is however recognised by the present inventors to be particularly harmful to the skin and a problem which should also be alleviated wherever possible.

This contact with the skin, and the presence of urine and faeces together, is harmful to the skin since this combined presence results in a chain of events causing breakdown of the skin. Faecal bacteria namely have urease which will react with urea from urine to thereby create ammonia. The presence of ammonia in the diaper will, in turn, raise the pH value and cause faecal protease and lipase to become more active, this activity increasing further with increasing pH. It is this activity which contributes to the wearer's skin becoming progressively and cumulatively damaged and leaves it at far greater risk to infections.

As will be apparent from the above, this is one of the reasons why nappy rash (diaper rash) and skin infections occur in body areas covered by absorbent products. Thus, whilst visual checks are made on the wearers of such products at specific or random intervals, the products in some cases (e.g. when simply wet) may in fact be less harmful even when left for long periods as compared to when urine and faeces are present together even for only relatively short periods.

Although some of the systems described in the aforementioned prior art documents are sensitive to wetness by urine, a need is present for an indicator means which is able to provide a more controlled transport of fluid, or transport of other information by means of a fluid, from a sensing or detection location to an indication location of said absorbent product. Similarly, an indicator means is needed which can more specifically indicate the presence of specific substances and/or specific properties of such substances in absorbent products.

It is also preferred if the indicator means can be constructed in a way which helps to prevent false detections.

The present invention is aimed at solving these problems.

By providing such a means, this can be used to further reduce the possibility of skin damage, based on the reliable recognition of harmful conditions within an absorbent product, to thereby allow said product to be changed when such conditions arise, and not at too early or late a stage.

It is a secondary object of the invention that the means should be cheap to both manufacture and to include in an absorbent product, such that the required means can be unitary with a disposable absorbent product.

### Summary of the invention

The main objective is achieved by an absorbent product having the features defined in claim 1. Preferred features of the invention are defined in the dependent claims.

Further features of the invention will be apparent to the reader of this specification.

In accordance with the invention, an indicator means is provided which has at least a part thereof which functions as an indicator portion to provide a signal to the wearer or an attendant that a particular condition (e.g. wetness) exists within the absorbent product. The transport means of the indicator means transports or transmits the particular condition in the product to the indicator portion in some way. This may be the transport of the exudate substance itself towards the indicator portion or it may for example be transporting some signal relating to the property of the exudate, such as its pH value, or simply the H⁺ ions responsible for said pH value. Similarly, other properties of the exudate may be transported via the transport means, such as temperature.

In order for the transport to occur in the present invention, fluid is used, the fluid either being from the exudate itself (e.g. urine) or from a further substance positioned in the indicator means or in the absorbent product which can react with the exudate to provide a fluid, or even from a fluid which is pre-stored in the indicator means (e.g. in the hollow fibres). Other possibilities also exist and can be used in conjunction with this invention.

The fluid uses a transport means in the form of a hollow fibre or fibres to reach the indicator portion. A hollow fibre is intended to mean a fibre which is not solid, but which is provided with an internal bore (capillary) in which fluid may exist. Thus, fibres which have their internal bore pre-filled with fluid or some other substance (e.g. gas) are to be understood as being comprised within the invention.

The hollow fibres of the invention need to be able to communicate with the indicator portion. Thus, one end of the fibre (or fibres) must be connected to the indicator portion. Such a connection with the indicator portion may be made directly via one open end of a hollow fibre, or the open end may be connected indirectly via a further means, such as an intermediate substance (e.g. a dissolvable substance) positioned between the end of the fibre(s) and the indicator portion.

Where transport of fluid itself along the hollow fibres should occur, the fibre capillary must be chosen to provide adequate capillary pressure so that the required fluid can be transported from the detection location to the indicator portion. Fibres which can be used with the invention are for example hollow rayon fibres, hollow glass fibres, or hollow fibres of polyacrylonitrile (PAN), polyethyleneteraphthalate (PET), refined cellulose, polyester or nylon. Further possibilities also exist and these will be apparent to a person skilled in the art.

When applied in absorbent products, the hollow fibres should have a high critical surface tension of wetting. Polyacrylonitrile has a critical surface tension of about 50 whilst nylon has a critical surface tension of about 44 and polyethyleneteraphthalate has a critical surface tension of about 43. This makes them more suitable than hollow fibres of polyethylene for example, since polyethylene has a critical surface tension of only about 31.

Such hollow fibres exhibit a relatively constant internal cross-section (i.e. capillary) through which fluid may be transported and thus a relatively constant speed of fluid movement can be obtained. The precise speed of fluid movement can be decided based on the type of fibre to be used and the size of the capillary and the type of fluid to be transported. Suitable fibres which could be used in absorbent product applications are for example hollow polyester fibres of 500 micron diameter with a wall thickness of 20 microns. Such are known products and can be obtained for example from the company Acordis.

Further suitable fibres may be those supplied by Dupont, who produce the well known Hollofil® and Quallofil® fibres. Still further possibilities which can be used are Thermolite® fibres (these being single-hole recycled preconsumer polyester fibres). Since Hollofil®, Quallofil® and Thermolite® fibres are all hypoallergenic, this makes their use in absorbent products (which are used in contact with the skin) particularly suitable. A still further possibility from Dupont is Antron Excel fibres, which are 6.6 Nylon (polyamide) multiple hole nylon fibres.

Similarly, the length of the hollow fibres should be sufficient to be able to reach from the point at which a detection of some type is being made up to the direct or indirect connection to the indicator portion. Depending on the required delay time or fluid volume required to produce an indication, the capillary and length of the fibre(s) can be adjusted accordingly. For a shorter delay time, the capillary can be chosen to be smaller for example.

The other end of the hollow fibre(s) remote from the indicator portion may be simply open, so as to allow fluid such as urine to enter the hollow fibre and then be transported to the other end of same. However, this remote end may also be covered with a substance preventing immediate entry of fluid, which substance may be for example dissolvable due to contact with urine or water. Such a means is herein referred to as a gate means and will generally be caused to "open", in particular by changing its state from a first state to a second state, as a result of its contact with body exudate. For example, the gate may be arranged to change state by urine or faeces being in direct contact therewith. However a product of the exudate, such as faecal gas (e.g. containing mercaptans, amines and other gases) or ammonia etc., can alternatively and/or additionally be used to operate on the gate to change its state.

The indicator means of the invention could further comprise a detector portion such as a sensor within the absorbent product, connected by the transport means to an indicator portion such as a visible or audible sign outside the absorbent product. The indicator portion is spaced from the detector portion by the transport means, whereby the aforementioned gate means could be arranged to prevent activation of either the detector portion and/or of the indicator portion and/or of the transport means.

As an example, if the gate means is a dissolvable substance, it could be coated on the detector portion so as to prevent contact of exudate with the detector until it has been in contact with the gate means for a predetermined time.

The gate means itself is normally in its first, passive or rest state but changes upon a predetermined threshold being exceeded. The threshold chosen depends on the parameter being evaluated for deciding when an indication to an observer should be provided.

The parameter used could be contact time (e.g. time within which urine, faeces and/or a gas resulting therefrom affects the gate particularly by contact therewith), a pH threshold value, or any other chosen parameter. Similarly, a combination of parameters might be used, such as pH and time combined, whereby a predetermined pH level would have to be reached and maintained for a certain predetermined time before the gate would change from its first state to its second state.

In this way, an indication can be provided based on a predetermined set of conditions. which allows an absorbent product designer the possibility of varying the predetermined gate parameters so as to suit different groups of user and size of product.

As mentioned in the introduction, the pH value of urine present in a diaper can be a particularly important factor for determining the presence of undesirable conditions. This pH value can be detected directly by allowing urine to come into contact with an indicator substance by means of the transport of the urine to an indicator portion. However, the H⁺ ions might be transported by providing a hollow fibre which is pre-filled with a fluid such as water, along which the H⁺ ions may flow from a source thereof to an indicator portion by means of diffusion. The source may be either from the exudate itself or from a product in one of the absorbent product layers, or from an exudate-activated source somewhere in the indicator means.

If pH is the parameter chosen to determine when the gate is to switch to the second state, a selected pH threshold level could be predetermined, such as a level of 6.5 or 7.0 or above, before the exudate (or product thereof) is able to activate the indicator means.

To avoid too rapid an indication for the case where only a small quantity of urine of high pH value is present (which may pass through the bodyside layer and into the absorbent core in a matter of seconds) a predetermined time factor can be built into the gate. For instance, taking a gate made of a substance which is arranged to break down only after a specific pH level is reached (e.g. a pH-sensitive gate terpolymer or other dissolvable substance such as a mildly acidic salt, chitosan, etc., or other substances presenting some change of state when in contact with a fluid having a pH-level higher than a predetermined threshold), the time aspect can be accounted for by adjusting the thickness of the terpolymer (or the other substances). Thus when the predetermined pH threshold is exceeded, the terpolymer gate, for example, starts to dissolve but further requires contact with the urine for a specific (selected) time (e.g. several minutes) before dissolving to allow urine to flow along the hollow fibre transport means to allow activation of an indicator portion. In this way, if the urine is of such a quantity that it is absorbed into the core and thus kept away from the terpolymer gate, the gate will remain sufficiently intact to prevent activation of the indicator means, whilst when sufficient urine is present around the area of the gate (i.e. due to a large amount of urine present), the gate means will be continuously broken down by the urine so as to allow the indicator means to be activated when the selected time has elapsed.

Additionally, it is possible to provide a plurality of hollow fibres connected to a plurality of indicator portions. In this way, one or more fibres would be connected to one indicator portion and other fibre(s) would be connected to other indicator portions. Similarly, each of these fibres may be connected to a different type of gate means. In this way, a parallel transport would be effected. If desired, the gate means can be used as the factor determining the actual threshold to be overcome before an indication can occur, while the hollow fibre itself only need have the function of transport (e.g. of urine). For example, if urine is to be transported from an absorbent core to separate indicator portions in the waist of a diaper, a first fibre capillary gate may be opened at pH 6.5 and a further one in a parallel hollow fibre opened at pH 6.7 or pH 6.9 or a higher pH level. In fact, these processes could happen virtually simultaneously if the pH is already at pH 7.0 or higher. Alternatively, only one might be opened if the pH is at 6.5, but the second one opened later if the pH were later to rise to a higher level (e.g. pH 7.0). Similarly a first indication might be made after urine of, for example, pH 7.0 or above is present for one minute followed by a separate indication when urine of pH 7.0 or above is present for ten minutes. These separate indications might be made by different colour indicators becoming visible.

Thus, an attendant could be warned by the first indication that a diaper change is merely "advisable" and by the second indication that a diaper change is "important".

Where a plurality of hollow fibres are arranged in parallel in the absorbent product, the spacing between the hollow fibre outer surfaces should be chosen to be large enough so as to prevent urine from being transported by any capillary action between the fibres. In this way the transport of fluid will be restricted to a transport path in the fibre capillaries which assists in preventing exudates erroneously reaching the indicator portions connected at the ends of the fibres.

Where gate means are present they may also be arranged in series along the path from the point of detection (e.g. at one open end of a hollow fibre) to the point of indication (e.g. at the other end connected to an indicator portion).

In certain embodiments, it is imaginable that more than two indicator portions can be used and different types of gates may be used for detecting different exudates or other parameters.

A hollow fibre indicator means of the invention may also be used together with another type of indicator means.

It will be appreciated that the present invention may find particular use in absorbent products where the exudates urine and faeces are most commonly present. Such absorbent products include especially diapers (for adult, infant and baby use), absorbent pants having an absorbent core (e.g. so-called training pants) as well as diaper chassis members and other incontinence products which have replaceable absorbent inserts. The invention may however also find application in various other types of hygiene articles, such as sanitary napkins and the like, whereby for example menses may be transported via the hollow fibre(s) to an indicator portion, or whereby menses may be used to trigger the activation of an indicator portion in another way.

### Brief description of the drawings

The invention will now be explained in more detail with reference to certain non-limiting embodiments thereof and with the aid of the accompanying drawings, in which:
- Fig. 1: shows a plan view of a diaper having a transport means with a detector and gate means at one end, whereby the liner has been removed,
- Fig. 2: shows a cross-sectional view on to line II-II of Fig. 1,
- Fig. 3: shows an outline view similar to the product of Fig. 1, in which the transport means are arranged on top of the absorbent core and the indicator portion outside the upper edge of the diaper,
- Fig. 4: shows an indicator means, in which a gate is positioned between a detector portion and the transport means,
- Fig. 5: shows an indicator means with a dye encapsulated by a gate means,
- Fig. 6: shows a cross-sectional view through a diaper in the flat state depicting an indicator means with an externally located indicator portion,
- Fig. 7: shows an embodiment of parallel transport means with associated gates and indicator portions,
- Fig. 8: shows an embodiment of a transport means divided into separate parts and associated with gate means in series with a single indicator portion, and
- Fig. 9: shows an embodiment of a transport means with a staggered set of hollow fibres.

### Detailed description of preferred embodiments:

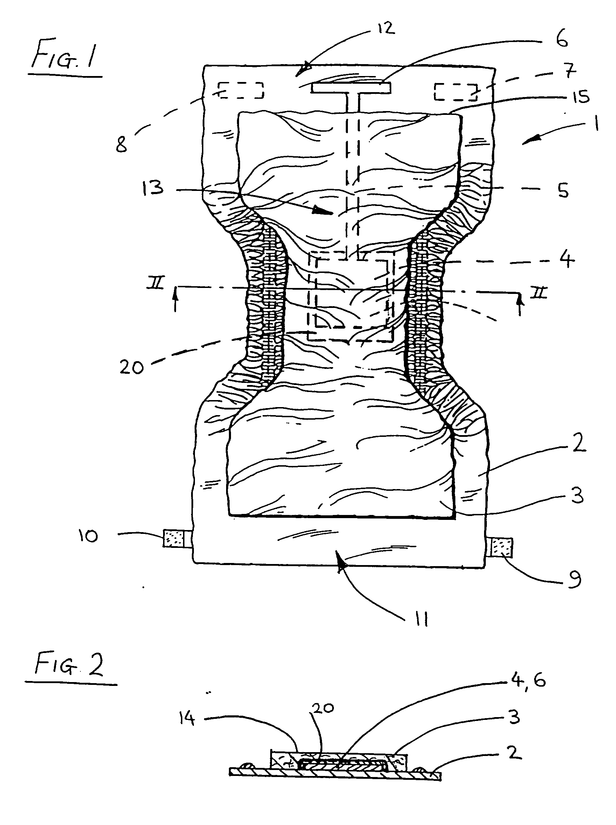

In Fig. 1, the absorbent product 1 in the form of a diaper has been illustrated in a flattened form and is depicted from the inner side thereof. For reasons of clarity, the top or cover sheet, sometimes referred to as a bodyside liner, has been removed.

Reference numeral 2 denotes a back sheet of liquid-impermeable material, typically consisting of a semi-transparent PE film. Such back sheet materials are common in the field of current day diapers and allow the underlying elements to be visibly discerned to a certain degree.

An absorbent core 3 of, for example, cellulosic material such as wood pulp fibres or the like, typically in an hour glass form, is positioned between the top sheet (not shown) and the back sheet 2. The absorbent core may be enveloped by a thin pervious layer such as a non-woven sheet (not shown) in some cases. The top sheet and back sheet 2 are then sealed around their contacting peripheries, preferably by means of welding or adhesive, to thereby form an envelope enclosing said core 3 and, if present, its enveloping pervious layer. The core 3 thus normally has dimensions which are smaller in both the length and width directions of the article, as shown.

Attachment means, such as hook and loop type mechanical fastening means, are provided at respective waist ends 11 and 12 of the article, whereby end 12 is intended to be the front waist portion of the product 1 and end 11 the rear waist portion, when applied to the wearer. In such an arrangement the tabs 7 and 8 could for example form hook tabs which are placed on the outer side of the back sheet 2, whilst tabs 9 and 10 could be loop tabs designed to engage with respective tabs 7 and 8 after the product has been passed through the legs of a wearer and up to the waist for attachment around the waist as is normal in the art.

An indicator means is attached to e.g. the back sheet 2 of the diaper and is denoted generally by numeral 13. As further shown in Fig. 2, the indicator means 13 has one end thereof, forming a detector portion 4, placed in contact with the absorbent core 3 in a generally centrally located portion, typically the crotch portion. The opposite end of said indicator means 13 comprises an indicator portion 6, shown lying at the front waist region 12 of said product 1, outside the end edge 15 of the core. A transport means 5 is coupled to the indicator portion 6 and joins same to the detector portion end of the indicator means. The transport means is in the form of one or more hollow fibres, through which some type of signal may be delivered from a point of detection to a point of indication.

The entire indicator means 13 is positioned on the inner, core-facing side of the back sheet 2 in this preferred embodiment. However, the portion of said means 13 which is within the core 3 may be situated away from the back sheet 2, for example at a location a few millimetres below the upper surface 14 of said core 3.

Surrounding the detector portion 4 is a gate means 20 in the form of a layer or coating of e.g. dissolvable material. As will be described below, such a gate means 20 could be constituted by a terpolymer coating, but other dissolvable layers may be used instead, possibly in addition to a terpolymer.

The detector portion 4 is thus positioned in a part of the absorbent core most likely to be affected by exudate, whereby the gate means prevents access of the exudate to the detector until the gate means has changed state (in this example by the gate means dissolving sufficiently for the exudate to contact the detector portion 4). A suitable location for the detector portion 4 and gate means 20 may thus be in the crotch portion of the absorbent product, close to the middle portion thereof (as shown). In this way, urine will have a high probability of reaching the gate means 20 and also later the detector portion 4 once the gate means has opened. Once urine or other substance has opened the gate means, the detector portion can send a signal of some type to the indicator portion 6, by means of the hollow fibre transport means 5.

As a general rule, the detector portion will be located inside the region of the absorbent core, even if not always at its centre, whereby the detector will be able to be proximate to exudate. Differences of detector location may occur between male and female absorbent products. The "region of the absorbent core" should be understood as being the general vicinity of the absorbent core. Thus, a detector portion 4 and gate means 20 located totally inside the absorbent core will clearly be in this "region", but also placement of the detector on one of the surfaces of the core or on a top or back sheet, and thus immediately adjacent to the core and within the plan view outline of the core, is intended to be encompassed by the expression "region of the absorbent core". The detector portion of the present invention may even be constituted by an end of a hollow fibre of the transport means located remote from the indicator portion.

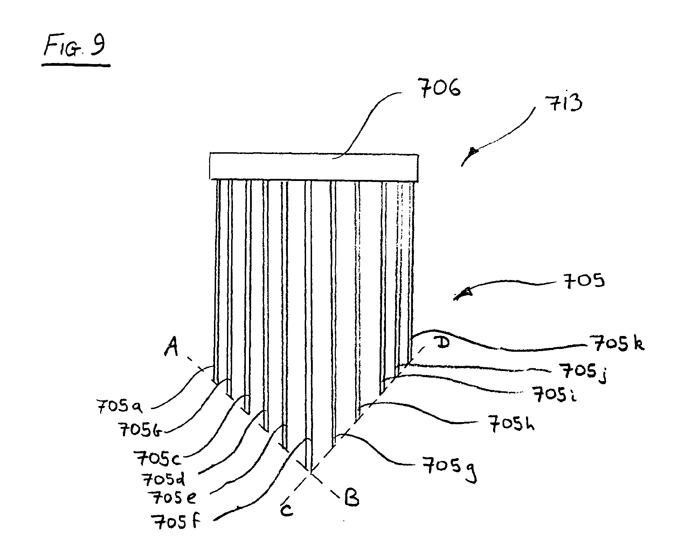

The detector portion and its associated gate means 20 could of course be given other shapes and forms and be located at another part of the core or even at several different parts of the absorbent core, to provide more probability of urine reaching the detector portion 4. Such an embodiment will be described below in connection with Fig. 9 for example. Similarly, whilst the hollow fibres are shown in the various embodiments as being straight, this is not a requirement and they may adopt different paths through the layers or absorbent core. Indeed one of the advantages of the use of hollow fibres for transport of urine, is that placement of the fibre in the core 3 itself is not problematic as regards the possibility of the fibre(s) becoming wetted by urine, since the outer side of the fibres will generally be water/urine impermeable and thus very small amounts of urine appearing for example midway between the detector and indictor portions (such as might occur with slight male incontinence) need not result in an indication being made.

As will be explained further below with respect to parallel transport means, there is also no requirement to have a separate detector portion per se, but instead one end of the transport means 5 may simply be open to exudate or open apart from a protective gate means.

The gate means 20 is shown as covering the detector portion 4, although it will be evident that for the gate means to operate, it must merely be able to prevent activation of the indication means 13 until required. Thus, the gate means could equally be placed anywhere in the path starting with the detector portion 4 (if present) right up to the indicator portion 6, or indeed over at least part of the indicator portion 6 itself.

The indicator portion 6 is intended to comprise any type of means that can provide a detectable indication, such as a visual indication, an audible indication, an indication by means of a specific fragrance, or even an indication by means of change of shape (e.g. for detection of a change of shape by blind persons), or even a combination of these indications.

In a preferred embodiment, in view of providing an indicator means which is cheap and can thus be used for mass production in disposable absorbent products, a visual indication is deemed most preferable. Thus, in the embodiment of Fig. 1, the indicator portion 6 has been shown positioned in a relatively easily visible position on the diaper, approximately midway between the strips 7 and 8, where it is unlikely to be obscured by the fastening of strips 9 and 10 thereto. However, the indicator portion may be positioned at any suitable location, such as close to the crotch portion of the article, although it will be appreciated that such a location may make inspection of same somewhat more burdensome, but at the same time it would have the advantage of being cheaper and smaller overall than the indicator means shown in Fig. 1. Similarly the indicator means could have at least the indicator portion 6 thereof positioned outside the wearer's normal clothing, in which case it would be attached by the hollow fibre(s) to the detector.

The transport means 5 starts to activate the indicator portion 6 upon the detector portion 4 detecting a required parameter. This start of activation can be controlled by the change of state of a gate means 20.

Where pH level is used as the gate means threshold, the indicator means preferably has a gate system able to detect, selectively, pH of specific threshold values. For example, a selective pH of substantially 6.5 and above in the exudate would not be susceptible to values below about 6.5. Where the detection of urine and faeces together is desired, higher pH values of for example about 6.7 or above, 6.9, 7.1, 7.3 or 7.5 or above respectively, might also be used as the detection threshold, since the higher thresholds are more easily distinguishable from some people's urine pH value which can be present dependent on the condition of the wearer.

A preferred gate means 20 is a terpolymer. Terpolymers are used traditionally to provide enteric coatings for pharmaceutical compositions which can be broken down by intestinal fluids (see e.g. US-A-4 704 295). Terpolymers are also used in the field of detergent compositions for cleaning (see e.g. US-A-5 000 689). However it has now been appreciated by the inventors that terpolymers can be usefully employed in the present invention as a gate means which can change state (i.e. by being dissolved/becoming permeable) so as to provide a threshold of pH and thereby an accurate indication of pH.

Terpolymers basically comprise three components, a temperature sensitive component, a polymer backbone (i.e. filler) and a pH sensitive component. In particular, terpolymers are formed by acid-ionisation such that they remain insoluble at lower pH values, depending on the ionisation level. This feature is useable in the present invention, since the lowest pH at which the terpolymer dissolves can be arranged within fine limits to a specific pH value. The techniques for fixing this pH value or set of pH values are readily known in the art of terpolymer construction and thus require no detailed description here.

In the embodiment of Figs. 1 and 2, the indicator portion 6 of the indicator means 13 may be constituted by a colour changing strip which is visible through the back sheet 2, whereby when the gate means 20 is broken down, urine may be concentrated in the detector portion and thus flow along the fibre(s) of the transport means 5 to the indicator portion 6.

The detector portion 4 is arranged to cause activation of the indicator portion 6 by sending a signal of some type (e.g. a colour signal, electrical signal, fluid signal etc.) by means of the transport means 5, whereby the gate means 20 would have to be placed in a suitable position to block said signal.

The transport means 5 hollow fibre(s) may be empty originally or it/they may be pre-filled with fluid. For example, the transport means fibre(s) may be filled with a pH indicator solution which, upon contact with urine will change colour as the urine diffuses into the indicator solution.

The transport means can be made opaque or transparent, whereby a transparent means would allow visibility of a changed indicator solution along the length of the fibre(s) and in the indicator portion 6, whereas opaque fibre(s) might only allow visibility of a changed colour solution at the indicator portion itself.

The indicator portion 6 may be constituted for example by a thin, impermeable transparent pouch also filled with indicator fluid and connected directly to the open ends of the transport means 5 fibre(s).

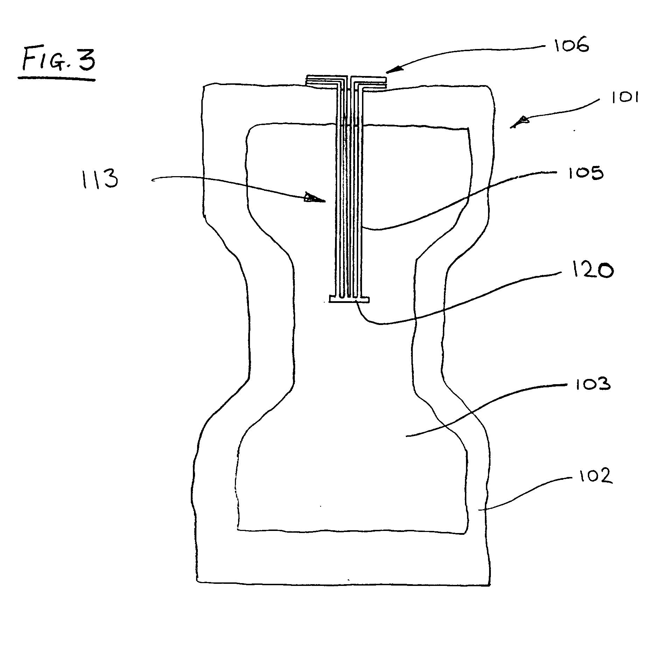

Alternatively, an indicator portion 6 may be formed by the end portions of the transport means 5 fluid-filled fibres themselves, whereby these may suitably be made to lie along the waist portion of the article. Such an embodiment is shown in Fig. 3, which is a simplified schematic view of the article shown in Fig. 1, whereby the indicator means 113 is shown positioned entirely on the bodyside of core 103. However the principle illustrated in Fig. 3 is not to be understood as being limited to an indication means lying on the bodyside face of the core.

The article 101 thus comprises a transport means 105 including four hollow fibres (although more or less can be used) each with its outermost end closed to prevent fluid loss. Each of the end portions of these fibres lies outside the outline of the diaper in a position allowing easy access/viewing. The four end portions constitute the indicator portion 106 of this embodiment. At the opposite end of the indicator means 113, the open ends of the fibres are sealed by a gate means 120, which can be broken down to allow urine or other exudate to access the indicator solution in the hollow fibres.

This has the advantage that no sealing means is required at the connection of the transport means and the indicator portion, since the indicator portion is formed by a continuous extension of the hollow fibres.

A terpolymer arranged to dissolve at pH of e.g. about 6.5 and above, could be used as a suitable gate means 20.

The gate means may also be in the form of a substance covering the indicator portion 6, which gate means is opaque until a certain threshold is reached and then changes state to be transparent, thereby providing visual access to a visible indicator underneath the gate means.

Where empty hollow fibres are used, urine which is released by the wearer into the region of the detector portion 4 may be drawn along the hollow fibres of the transport means 5 by capillary action, as explained previously. When the urine reaches the indicator portion 6, the urine may act directly on the indicator portion 6 to e.g. change its colour. Alternatively, the urine may act on a dissolvable coating (e.g. a terpolymer) initially obscuring the indicator portion 6 from view. In this way, a visible indicator substance (e.g. a fluorescent strip) can become visible from beneath the terpolymer coating when a required pH level is exceeded. Additionally or alternatively to the gate means changing state to allow the underlying indicator to become visible, the indicator means 13 could itself also be colour changeable at or above the required pH, and protected entirely by the gate means in the form of a covering layer (e.g. the aforementioned terpolymer). In this way, the gate means could provide a first barrier to the indicator portion being visible and, when the gate changes state (e.g. the terpolymer dissolves) as a result if its contact with exudate, the colour changeable indicator could be allowed to come into contact with the exudate substance having a pH of e.g. 7.0 and above and thus change colour. This could be used to provide a dual-level indicator system, whereby the covering layer gate means (terpolymer) breaks down and dissolves at e.g. pH 6.5 to reveal the underlying indicator portion 106 in one colour, whereas only when the pH rises to 7.0 or a higher value, the indicator can assume a further colour which is also visible. In this way, the wearer or attendant can be warned at a first pH level (caused by the terpolymer dissolving for example) that harmful conditions exist and then at a second level (caused by the indicator portion 6 colour change) that the pH has risen to a new higher level (e.g. 7.0 or 8.0) such that rapid degradation of the skin is likely to occur unless the absorbent product is changed soon.

The thickness of the gate means 12, 120 depends on the predetermined time threshold which is desired before visual indication should take place after the parameter threshold has been exceeded. This may be used advantageously so as to avoid warning the wearer or attendant of a urine pH value which is possibly high and undesirable, but which at the same time is so small that it is entirely absorbed in the lower part of the core 3 (i.e. part closest to the back sheet 2 during use) and thus of relatively insignificant danger for the wearer's skin. A thicker layer will evidently take more time to dissolve than a thinner layer. Thus, where the time threshold is wished to be very low (e.g. a few seconds), or substantially zero, only a very thin coating of terpolymer or other dissolvable substance is necessary. Similarly, as mentioned above, the length and/or capillary of the transport means 5, 105 can also be altered such that a greater or lesser time is required to transport fluid to the indicator portion (or to a gate means preceding the indicator portion).

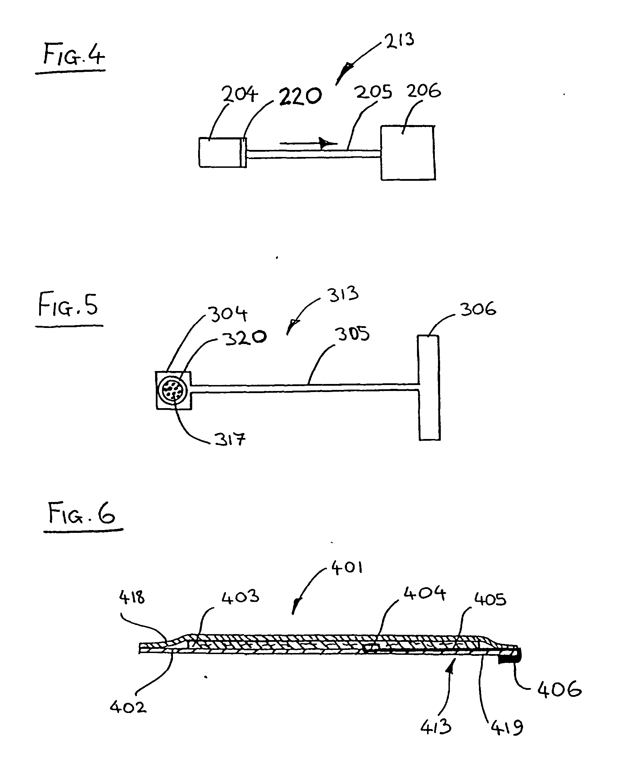

Fig. 4 illustrates a sketch of a further possibility whereby a gate means 220 (formed by a terpolymer or another threshold-sensitive means) prevents any signal produced by the detector 204 from reaching the hollow fibre(s) of the signal transport means 205, to thereby inhibit the indicator portion 206 from being activated to produce an indication. For example, detector 204 may provide a source of coloured dye that is to be transported to indicator portion 206 by means of the hollow fibre(s) of the transport means 205 (e.g. hollow fibres pre-filled with water), whereby the gate 220 first needs to be changed to its second state to allow movement of the dye down the means 205. As soon as the gate is in its second state, coloured dye can be released into the fluid-filled hollow fibres and will migrate along these and fill the indicator portion 306. Alternatively, detector portion 204 may comprise a urine collection device (e.g. a one-way membrane) and the urine itself may be used to travel up an empty hollow fibre to cause an indication at the indicator portion 306.

Fig. 5 shows an embodiment similar to Fig. 4, whereby a coloured dye 317 is stored in an encapsulation of a pH sensitive blocker material constituting a gate means 320 as part of detector portion 304. Upon breakdown of the blocker material of the gate means 320 (e.g. a terpolymer), the dye 317 is allowed to travel along fibre(s) of the transport means 305 to thereby be displayed by the indicator portion 306. The detector portion 304 and the connector line 305 can thus be made of an opaque material, whilst leaving the indicator portion 306 transparent.

As an alternative (not shown), the dye 317 might be replaced by, or supplemented with, a foam substance which when activated due to the gate means changing to its second state, causes foam to be formed at a fast rate and thereby to pass along the hollow fibres of the transport means and fill an expandable cavity in the indicator portion. In this way, a change of shape can constitute the indication, whereby the indicator means 306 would be placed in a location where the change of shape could be easily felt by the user. This may be suitable for users who are colour-blind or have defective sight. For example, the indicator means may provide a smooth surface having weakened expandable portions in the normal state, whereby filling the indicator means 306 with foam or gas or the like (as a result of pH above 6.5 for example) would expand said weakened portions and thus change the texture of the smooth surface so as to be recognisably different (visually and/or by touch) to the surface in its original state.

Alternatively, the exudate or another substance within gate 320, may be arranged to flow along the hollow fibre(s) of the transport means 305 until reaching an indicator portion 306, in which a gas or foam releasing agent (not shown) is stored.

Although a terpolymer has been proposed as a particular embodiment of a gate, other possibilities exist. A further possibility is for example a pH-sensitive hydrogel. Carboxylated hydrogels are particularly suitable since they are non-toxic, cheap and present a sharp pH shift, thus allowing high accuracy pH to be used as a threshold value for the gate. pH-sensitive hydrogels change state by swelling in such a way that they become permeable to both small and large molecules. The critical value of pH at which a hydrogel will swell is dependent on the pKa value of the acid moieties of the hydrogel. In this way, the skilled person can provide a pH threshold value adjusted to swell at the required pH.

Thus, taking the embodiment of Fig. 4 for example, the gate 220 can be formed by a hydrogel which initially is plugged by large molecules which are freed, at the required pH, due to the swelling of the hydrogel. The freeing of the large molecules opens one or more channels through which the contents of part 204 comes into contact with the contents of part 205. This can likewise be applied to the embodiment of Fig. 5 by construction of the gate 320 as a hydrogel carrying large molecules which upon swelling are freed to open channels allowing dye 317 (or other component) to pass through the gate 320.

A further alternative for a gate is to use any one of many known mildly acidic salts which will saturate at low pH levels and dissolve freely in alkali conditions. Depending on the pH dissolution threshold level required, it is merely a matter of selecting a salt e.g. from the Merck Index listing of strong acids and weak bases (e.g. calcium sulphate salt or the like). This selection is not described in further detail here since the selection of specific salts is not a limitation of the present invention. It may also be mentioned that there are also any number of low-molecular weight organic acid salts which are readily available having a wide range of dissolution points.

A still further possibility is the use of chitosan as the gate material. Chitosan is namely soluble in dilute aqueous organic acids (thus including values for example pH values between 6.5 to 7.0) and thus applicable for use as a gate in applications of this invention. Chitosan is namely not soluble in many other solutions.

Although dissolution of the gate of blocking material upon a required threshold value being reached has been described in some of the above embodiments, further possibilities include constructing the gate from a compound which exhibits a marked change in viscosity at a particular threshold of pH. For example, proteins have no significant shift in solubility as pH varies since they are highly amphoteric. However, proteins do show a shift in viscosity value or gel point with pH which can be sufficient for this invention. For example, taking the embodiment of Fig. 4, the line 205 may be constructed as one or more hollow fibres filled with fluid, which fibre(s) can be plugged with a highly viscous polymeric gel which becomes less viscous at the required pH (e.g. at pH 6.5 and above, 6.7 and above, etc.). This then allows the plug to be freed at the required pH (i.e. the gate is opened). The selection of the requisite polymeric gel is not described in further detail here, since the specific polymer can simply be selected according to the pH value required.

Fig. 6 shows an arrangement of an indicator means 413 having a detector portion 404, transport means 405 and indicator portion 406. The indicator portion 406 lies on or adjacent to the external surface 418 of the back sheet 402 whilst the detector portion 404 and part of transport means 405 may be arranged between the top sheet 418 and back sheet 402, the detector portion being in or adjacent to the absorbent core 403. In this way, perception of the indicator portion is facilitated, be it audio, visual or a combination of any number of perceivable indications. The transport means 405 is shown as being the part which is curved around the back sheet 419, although clearly this should be done with such a radius of curvature that the fibre capillaries are not squashed or bent, which would inhibit transport. Alternatively, the hollow fibres may be allowed to remain straight and stop just inside the diaper edge whilst the indicator portion itself may be the part of the indicator means which is folded over the top edge of the diaper.

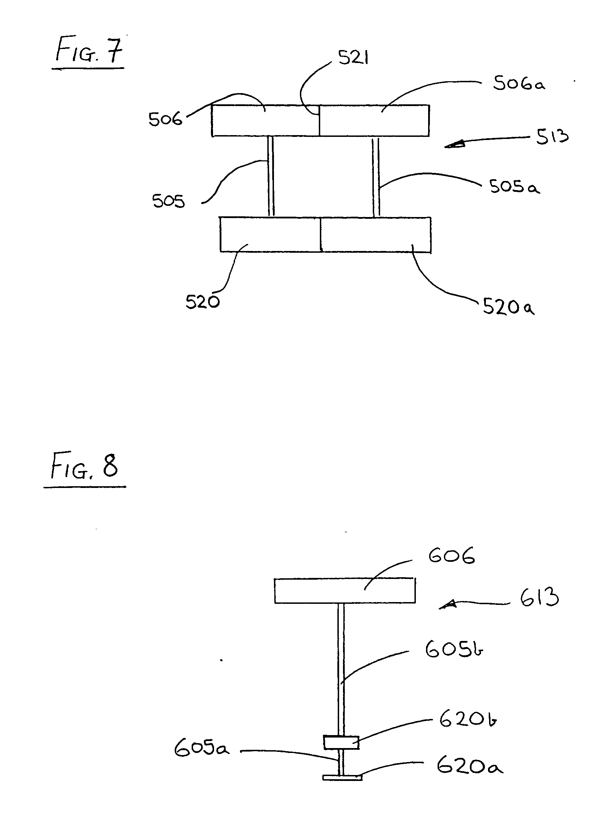

Fig. 7 illustrates an embodiment of parallel gate means 520, 520a connected by means of parallel fibres or sets of fibres in transport means 505, 505a to indicator portions 506, 506a respectively. The gate means 520, 520a may for example be placed in an absorbent core (not shown) and the indicator portions 506, 506a in a visible position on the absorbent product (e.g. at the waistband of a diaper). Indicator portion 506 may for example be red at pH values below about 5.0 and change to a blue colour at pH values of between about 5.0 and 8.0. Such an indicator could be a 2.5-dinitrophenol indicator. Similarly indicator portion 506a might be an indicator which changes colour from yellow to purple above about pH 4.8 and up to 6.4 (such as a cholorophenol red indicator). Other ranges may be chosen depending on what indication is required. Transport means 505, 505a may be used to transport urine to activate the indicator portions 506, 506a respectively when gate means 520, 520a have changed state. The gate means 520, 520a may each be constituted by a terpolymer and capable of dissolving at pH values of e.g. 5.5 and above and 6.5 and above respectively. Thus when gate 520 changes state by dissolving above the required pH threshold, it allows activation by urine of the first indicator 506 so that this will change colour to blue. If the pH is below 6.5, it will however not dissolve gate 520a and thus no colour change will occur at indicator 506a. In this way, an indication of wetness due to urine is made, but the pH level does not imply that an urgent change of absorbent product is needed. If however, the purple colour appears on indicator portion 506a, the attendant is warned that skin damage may occur if a change does not occur soon.

From the above it is clear that although the pH ranges set by the individual colour indicators themselves are relatively broad, the gate means 520, 520a is capable of providing a much greater level of accuracy in pH evaluation and thus better information to the attendant/wearer.

Although transport means 505 and 505a may be single hollow fibres, each can be replaced by a plurality of hollow fibres to allow connection of same with a larger proportion of the indicator portions 506, 506a.

While the above embodiment is shown with only two indicators in parallel, it should be understood that more indicators can also be used in parallel and/or in series. Thus, a whole range of gates and indicators could for example be provided at differing pH values, such as pH 5.3, 5.6, 5.8. 6.1, 6.3 etc, each indicator portion being provided with one or more hollow fibres coupled therewith, whereby values above for example pH 6.5 may be used to indicate the need for a product change.

Similarly, while the elements 506 and 506a are shown as joined in the form of a strip, the indicators do not need to be physically interconnected but could be separate. Preferably the indication area would be kept to a minimum and thus a small strip of indicator segments (i.e. similar to indicator portions 506, 506a) would provide a suitable means of display.

Furthermore whilst the indicators in the above embodiment have been described as being constituted by different colour changes, the same colour changes can be used (e.g. red to blue litmus indicator), whereby when the first gate means 520 changes state (e.g. at urine of about pH 5.8 for example) the portion 506 will turn blue, but the second indicator 506a will not change to blue until the higher specific pH threshold is reached. In this way, an attendant can also be informed of the urgency of an absorbent product change by seeing how much of the combined strip 506, 506a has changed colour. If the strip 506, 506a would be a paper strip, a separation means 521 of some type (e.g. an adhesive line within the strip) would be useful to stop one part of the strip affecting the other and thus effectively circumventing the gate means and transport means.

Figure 8 depicts an embodiment of an indicator means with two gate means 620a, 620b in series. The gate means 620a and 620b are joined by a first hollow fibre transport means 605a. Similarly, second hollow fibre transport means 605b joins gate means 620b to indicator portion 606. Gates 620a, 620b may be positioned appropriately in, or in the vicinity of, an absorbent core in a manner similar to that shown in Fig. 1 for example. Although shown in Fig. 8 as separating two separate sections of hollow fibre transport means 605a, 605b, the gate means 620b may however be placed within the capillary of a single continuous hollow fibre (or fibres) extending from gate 620a to indicator portion 606. A suitable location of same might be midway along the capillary.

Taking an exemplary embodiment of a series of gates, the gate means 620a may be in the form of a very thin terpolymer layer dissolvable within a few seconds of contact with a solution of a selected lower pH threshold (e.g. pH 6.5) to thereby open the transport means 605a to allow passage of liquid (e.g. urine) along its hollow fibre(s) to gate 620b. Likewise, second gate means 620b may be a substance (e.g. a further terpolymer) dissolvable after a specific pH level is exceeded, for example at a pH above 4.5 and preferably at a level lower than said first pH level. The thickness of the second gate means however can be chosen such that a selected time period (e.g. a number of minutes) is required before the gate means changes to its second state.

The choice of gate means can be chosen to suit the particular parameter evaluation that is required for activation of the indicator means.

If it is desired that the presence of urine be detected over a large area of the absorbent core, the transport means can be arranged in the form of a set of parallel hollow fibres whereby the ends of the hollow fibres remote from the indicator portion present a staggered configuration. Such an embodiment is shown in Fig. 9 for example, showing a plan view of an indicator means with an indicator portion 706 connected to a transport means 705. In this embodiment the transport means 705 comprises a series of hollow fibres 705a-705k which can be arranged such that, for example, the open end of fibre 705f lies in the centre of the absorbent core and the open ends of the other fibres lie at longitudinally staggered positions in the core such that fibres 705a and 705k would lie close to the laterally outer edges of the core. In order to produce such a fibre shape, a simple cutting operation along lines A-B and C-D would suffice. Alternatively, a single straight cut line A-B could be used, whereby cut line A-B would be extended in a straight line to cut all fibres 705a to 705k in a single cut. This would then also provide fibre open ends on both sides of the core centreline (positioned approximately at the intersection of lines A-B and C-D).

As will be apparent, the embodiment of Figure 9 may also be provided with gate means.

Further embodiments will be readily understood by the skilled person upon reading the aforegoing and are intended to be encompassed within the scope of the invention as defined by the appended claims. For example, while the embodiments described generally rely on the use of urine to provide an indication, other exudates can also be used to trigger the transport of a signal through the transport means hollow fibres.

## Claims

1. An indicator means (13; 113; 213; 313; 413; 513; 613; 713) for use with an absorbent product (1; 101; 401) for absorbing bodily exudate, wherein said indicator means comprises at least one indicator portion (6; 106; 206; 306; 406; 506, 506a; 606, 706), and wherein said indicator portion is connected to transport means (5; 105; 205; 305; 405; 505; 605a, 605b; 705) for transporting a signal to said indicator means, said signal being indicative of a condition within an absorbent product, **characterized in that** said transport means (5; 105; 205; 305; 405; 505; 605a, 605b; 705) comprises at least one hollow fibre, one end of which is connected to said at least one indicator portion (6; 106; 206; 306; 406; 506, 506a; 606, 706).

2. An indicator means according to claim 1, **characterized in that** said transport means (5; 105; 205; 305; 405; 505; 605a, 605b; 705) comprises a plurality of hollow fibres, each with one end connected to at least one indicator portion (6; 106; 206; 306; 406; 506, 506a; 606, 706).

3. An indicator means according to claim 1 or 2, **characterized in that** a plurality of indicator portions (106; 506, 506a) is provided, each of which indicator portions is connected to an end of at least one hollow fibre.

4. An indicator means according to claim 2 or 3, **characterized in that** said hollow fibres are spaced from one another by a distance sufficient to avoid fluid being transported as a result of capillary action between said fibres.

5. An indicator means according to any one of the preceding claims, **characterized in that** said at least one hollow fibre is a hollow polyester filament.

6. An indicator means according to any one of claim 1 to 4, **characterized in that** said at least one hollow fibre is a hollow glass fibre.

7. An indicator means according to any one of the preceding claims, **characterized in that** said at least one hollow fibre is pre-filled with a fluid, such as water, capable of transporting H⁺ ions by means of diffusion to said indicator portion (6; 106; 206; 306; 406; 506, 506a; 606, 706).

8. An indicator means according to any one of the preceding claims, **characterized in that** said at least one hollow fibre is provided with a gate means (20; 120; 220; 320; 420; 520; 620a, 620b) which in a first state prevents activation of said indicator portion (6; 106; 206; 306; 406; 506, 506a; 606, 706), and **in that** said gate means (20; 120; 220; 320; 420; 520; 620a, 620b) can change from said first state to a second state in which activation of said indicator portion (6; 106; 206; 306; 406; 506, 506a; 606, 706) is permitted.

9. An indicator means according to claim 8, **characterized in that** said gate means (20; 120; 220; 320; 420; 520; 620a, 620b) changes state at a predetermined pH threshold and/or time threshold.

10. An indicator means according to claim 8 or 9, **characterized in that** said gate means comprises a pH-sensitive terpolymer having a predetermined pH threshold.

11. An indicator means according to any one of claims 8 to 10, **characterized in that** said gate means is located at one end of said at least one hollow fibre.

12. An indicator means according to any one of claims 8 to 11, **characterized in that** a plurality of hollow fibres and a plurality of indicator portions (506, 506a) are present, and **in that** each of said indicator portions is associated with a separate gate means (520, 520a) having a different threshold from other gate means to thereby allow the indicator portions (506, 506a) to be activated at different thresholds.

13. An indicator means according to any one of the preceding claims, **characterized in that** a plurality of hollow fibres (705a-705k) is present, and **in that** the ends of said hollow fibres (705a-705k) remote from said indicator portion (706) present a staggered configuration.

14. An indicator means according to any one of the preceding claims, **characterized in that** said indicator means further includes a detector portion (4; 104; 204; 304; 404) remote from said indicator portion, and **in that** said at least one hollow fibre connects said detector portion (4; 104; 204; 304; 404) to said indicator portion (6; 106; 206; 306; 406).

15. An indicator means according to any one of the preceding claims, **characterized in that** the end of said at least one hollow fibre remote from said indicator portion (6; 106; 206; 306; 406; 506, 506a; 606, 706), is positioned in, or adjacent to, an absorbent core (3; 103; 403) of said absorbent product.

16. An indicator means according to any one of the preceding claims, **characterized in that** said absorbent product (1; 101; 401) is a diaper or a pair of absorbent pants.

17. An indicator means according to any one of the preceding claims, **characterized in that** said at least one hollow fibre has a capillary with a substantially constant cross-section.
